# EUROPÄISCHE PATENTSCHRIFT

(11) **EP 1 071 433 B1**
(45) Veröffentlichungstag und Bekanntmachung des Hinweises auf die Patenterteilung: **11.12.2002**
(21) Anmeldenummer: 99912962.0
(22) Anmeldetag: 14.04.1999
(51) Int. Cl.: A61K 31/70, A61P 37/06

(54) **2',2'-DIFLUORNUCLEOSIDE ZUR IMMUNOSUPPRESSIVEN THERAPIE UND KOMBINATIONSPRÄPARATE**
2',2'-DIFLUORONUCLEOSIDES FOR IMMUNOSUPPRESSIVE THERAPY AND COMBINED PHARMACEUTICAL COMPOSITIONS
2',2'-DIFLUORONUCLEOSIDES POUR LA THERAPIE IMMUNOSUPPRESSIVE ET PREPARATIONS PHARMACEUTIQUES COMBINEES

(30) Priorität: 14.04.1998 AT 63698
(43) Veröffentlichungstag der Anmeldung: 31.01.2001
(73) Patentinhaber: ELI LILLY AND COMPANY, Indianapolis, Indiana 46285 (US)
(72) Erfinder: MARGREITER, Raimund, A-6103 Reith b. Seefeld (AT); KONWALINKA, Günther, A-6020 Innsbruck (AT)
(74) Vertreter: Schwarz, Albin, Dr.
(86) Internationale Anmeldenummer: AT9900093
(87) Internationale Veröffentlichungsnummer: WO99052514

(56) Entgegenhaltungen:
- EP-A- 0 184 365
- EP-A- 0 328 345
- EP-A- 0 345 751
- EP-A- 0 576 227
- BIANCHI ET AL.: "Inhibition of ribonucleotide reductase by 2'-substituted deoxycytidine analogs: Possible application in AIDS treatment" PROC. NATL. ACAD. SCI. U.S.A., Bd. 91, Nr. 18, 1994, Seiten 8403-8407, XP002117871
- ALVINO ET AL.: "2'-2'-Difluorodeoxycytidine: In Vitro Effects on Cell-Mediated Immune Response" ANTICANCER RESEARCH, Bd. 18, Nr. 5a, 1998, Seiten 3597-3602, XP002117872
- DAIKELER T. ET AL: "The influence of gemcitabine on the CD4/CD8 ratio in patients with solid tumours." ONCOLOGY REPORTS, (1997) 4/3 (561-564)., XP002117873
- BORCHMANN P. ET AL: "New drugs in the treatment of Hodgkin's disease." ANNALS OF ONCOLOGY, (1998) 9/SUPPL. 5 (S103-S108)., XP002117874
- CORY ET AL.: "Effects of 2',2'-Difluorodeoxycytidine (Gemcitabine) on Wild Type and Variant Mouse Leukemia L1210 Cells." ONCOL. RES., Bd. 5, Nr. 2, 1993, Seiten 59-63, XP002117875
- VON HOFF D.D. ET AL: "Advances in the treatment of patients with pancreatic cancer: Improvement in symptoms and survival time." BRITISH JOURNAL OF CANCER, (1998) 78/SUPPL. 3 (9-13)., XP002117876
- BYRD J C ET AL: "Old and new therapies in chronic lymphocytic leukemia: Now is the time for a reassessment of therapeutic goals" SEMINARS IN ONCOLOGY, Bd. 25, Nr. 1, Februar 1998 (1998-02), Seiten 65-74, XP002104744
- MICHAEL M. ET AL: "Clinical experience with gemcitabine in pancreatic carcinoma." ONCOLOGY, (1997) 11/11 (1615-1622)., XP002117877
- GANDHI V. ET AL: "Modulation of arabinosylcytosine metabolism during leukemia therapy." ADVANCES IN EXPERIMENTAL MEDICINE AND BIOLOGY, (1995) 370/- (119-124)., XP002117878
- GRUNEWALD R ET AL: "Pharmacologically directed design of the dose rate and schedule of 2',2'-difluorodeoxycytidine ( Gemcitabine ) administration in leukemia." CANCER RESEARCH, (1990 NOV 1) 50 (21) 6823-6., XP002117879
- MALAYERI R ET AL: "Delayed anemia and thrombocytopenia after treatment with gemcitabine [letter]." JOURNAL OF THE NATIONAL CANCER INSTITUTE, (1997 AUG 6) 89 (15) 1164., XP002117880
- KOTRA ET AL.: "Structure-Activity Relationships of 2'-Deoxy-2',2'-difluoro-L-erythro-pentafur anosyl Nucleosides" J. MED. CHEM., Bd. 40, Nr. 22, 1997, Seiten 3635-3644, XP002117881
- CARMICHAEL J. ET AL.: "Advanced Breast Cancer: Investigational Role of Gemcitabine" EUR. J. CANCER, Bd. 33, Nr. suppl. 1, 1997, Seiten S27-S30, XP002117882
- VANDER ELS, N.J.; MILLER, V.: "Successful treatment of gemcitabine toxicity with a brief course of oral corticosteroid therapy" CHEST, Bd. 114, Nr. 6, 1998, Seiten 1779-1781, XP002117894
- KOTRA, LAKSHMI P. ET AL: "Structure-Activity Relationships of 2'-Deoxy-2',2'-difluoro-L- erythro-pentofuranosyl Nucleosides" J. MED. CHEM. (1997), 40(22), 3635-3644, XP000867642
- XIANG, YUEJUN ET. AL.: "SYNTHESIS AND ANTI-HIV ACTIVITIES OF 2'-DEOXY-2',2''-DIFLOURO-BETA-L-RIBOFURANO SYL-PYRIMIDINE AND PURINE NUCLEOSIDES." BIOORG. MED. CHEM. LETT., Bd. 5, Nr. 7, 1995, Seiten 743-748, XP002130446
- HERTEL, L. W. ET AL: "Synthesis, cytotoxicity and metabolism of the 2',2'-difluoro analogs of deoxyadenosine (dFdA) and deoxyguanosine (dFdG)" NUCLEOSIDES NUCLEOTIDES (1989), VOLUME DATE 1988, 8(5-6), 951-5, XP002130447
- CHEMICAL ABSTRACTS, vol. 124, no. 8, 19. Februar 1996 (1996-02-19) Columbus, Ohio, US; abstract no. 75742, ANDIS, SHERRY, ET AL.: "Medicinal chemistry of difluoropurines" XP002130448 & ANDIS, SHERRY, ET AL.: "Medicinal chemistry of difluoropurines" SEMIN. ONCOL., Bd. 22, Nr. 4 (suppl. 11), 1995, Seite 54-60
- CHEMICAL ABSTRACTS, vol. 124, no. 8, 19. Februar 1996 (1996-02-19) Columbus, Ohio, US; abstract no. 75743, GANDHI, VARSHA ET AL.: "Difluorodeoxyguanosine: Cytotoxicity, Metabolism, and Actions on DNA Synthesis in Human Leukemia Cells." XP002130449 & GANDHI, VARSHA ET AL.: "Difluorodeoxyguanosine: Cytotoxicity, Metabolism, and Actions on DNA Synthesis in Human Leukemia Cells." SEMIN. ONCOL., Bd. 22, Nr. 4 (Suppl. 11), 1995, Seiten 61-67,

## Beschreibung

### Gebiet der Erfindung

Die vorliegende Erfindung betrifft das Gebiet der Arzneimitteltherapie und stellt die neue Verwendung von 2',2'-Difluornucleosiden zur Herstellung von Zusammensetzungen zur immunsuppressiven Therapie und neue pharmazeutische Zusammensetzungen und Produkte zur Behandlung des menschlichen und tierischen Körpers zur Verfügung.

### Stand der Technik

Die Unterdrückung der Reaktivität des Immunsystems durch immunsuppressive Therapie ist von großer medizinischer Bedeutung bei der Verhütung der Abstoßung allogener Transplantate bei Transplantationspatienten und bei der Behandlung von Autoimmunerkrankungen. Im Laufe der vergangenen Jahre ist eine beschränkte Zahl neuer Medikamente, welche zur Anwendung in der immunsuppressiven Therapie geeignet sind, wie z.B. Cyclosporin A, Tacrolimus, Mycophenolatmofetil, Daclizumab und Rapamycin, entwickelt worden.

Nach wie vor besteht ein dringender Bedarf, wirksamere und besser verträgliche Methoden zur Behandlung von Autoimmunerkrankungen und für die Verhütung der Abstoßung allogener Transplantate bei Transplantationspatienten zu entwickeln. Die vorliegende Erfindung ist daher bestrebt, neue pharmazeutische Zusammensetzungen und Produkte zur Verwendung in diesem Therapiegebiet bereitzustellen.

Es ist gezeigt worden, daß 2',2'-Difluornucleoside in vitro antivirale Wirkungen (US-Patent 4,808,614) und in Krebs-Standardscreeningtests onkolytische Aktivität entfalten (US Patent 5,464,826). Von diesen Verbindungen ist das 2'-Desoxy-2',2'-difluorcytidin (Gemcitabin, dFdC) hinsichtlich seiner onkolytischen Aktivität umfassend untersucht worden (Kaye, *J. Clin*. *Oncol.* 12, 1527 (1994)). Auf der Grundlage der Ergebnisse dieser Studien erhielt Gemcitabinhydrochlorid in über 50 Staaten die behördliche Zulassung zur Behandlung von nicht-kleinzelligem Bronchialkarzinom und/oder Pankreaskarzinom. Weitere Studien zur Behandlung von Mamma-, Blasen- und Ovarialkaizinom mit Gemcitabin werden gegenwärtig durchgeführt.

### KURZFASSUNG DER ERFINDUNG

Die vorliegende Erfindung stellt die Verwendung einer Verbindung der Formel I 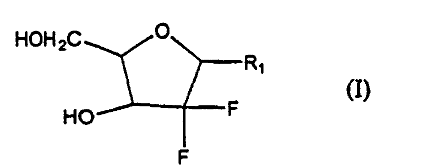 worin R₁ eine durch eine der Formeln 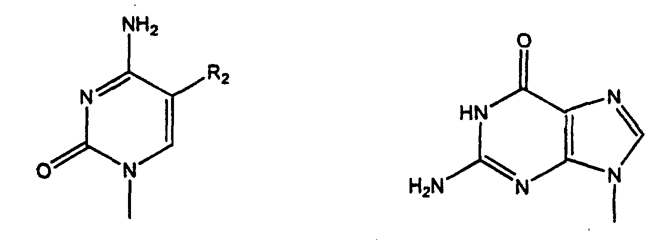 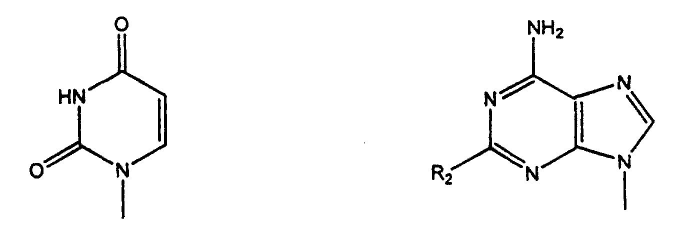 definierte Base ist und R₂ Wasserstoff, C₁-C₄ Alkyl, Brom, Fluor, Chlor oder Iod ist, oder eines pharmazeutisch annehmbaren Salzes davon zur Herstellung eines Medikaments zur immunsuppressiven Therapie des menschlichen oder tierischen Körpers zur Verfügung.

Die vorliegende Erfindung ist ferner auf die Verwendung einer Verbindung der Formel I zur Herstellung eines Medikaments zur Behandlung von Autoimmunerkrankungen bei Menschen und Tieren gerichtet.

Die vorliegende Erfindung stellt auch die Verwendung einer Verbindung der Formel I zur Herstellung eines Medikaments zur Unterdrückung der Abstoßung von Transplantaten bei Menschen und Tieren, vorzugsweise zur Unterdrückung der Abstoßung von Knochenmarktransplantaten, Herztransplantaten, Hornhauttransplantaten, Dünndarmtransplantaten, Lebertransplantaten, Lungentransplantaten, Pankreastransplantaten, Nierentransplantaten und Hauttransplantaten zur Verfügung.

Bei einem anderen Aspekt der Erfindung wird die Verbindung der Formel I zur Herstellung eines Medikaments zur Behandlung einer Krankheit oder eines Zustands verwendet, welche(r) ausgewählt ist aus: Rosacea, Acrodermatitis continua, Aktinisches Retikuloid, Alopezie, Alport-Syndrom, amyotrophische Lateralsklerose, Stomatitis aphthosa, Pure red cell aplasia, aplastische Anämie, Asthma, atopische Dermatitis, Autoimmun-Enteropathie, Behcet-Krankheit, bullöses Erythema exsudativum multiforme, bullöses Pemphigoid, Biliäre Zirrhose, Cornea-Schmelzsyndrom (engl.: corneal melting syndrome), Crohn-Krankheit, Dermatitis herpetiformis, Dermatomyositis, Diabetes mellitus, Duchenne-Form der Muskelatrophie, Ekzem, Epidermolysis bullosa, Erythema nodosum leprosum, familiäre hämophagozytische Lymphohistiozytose, Felty-Syndrom, Granuloma anulare, Grave-Ophthalmopathie, hämolytische Anämie, Hämophilie, Hepatitis, Ichthyosis, entzündliche Erkrankung des Darms (engl.: inflammatory bowel disease), interstitielle Cystitis, interstitielle Lungenkrankheit, Keratokonjunktivitis, Histiozytose der Langerhans-Zellen. Lichen planus, Makrophagenaktivierungssyndrom, Mooren-Ulcus, Morphaea, multiple Sklerose, Myasthenia gravis, Nephropathie, nephrotisches Syndrom, Pustulosis palmaris et plantaris, Pemphigus, persistierende Photosensibilität, Pityriasis rubra pilaris, Polymyositis, Psoriasis, Arthritis psoriatica, Lungenfibrose, Pyoderma gangraenosum, retikuläre erythematöse Mucinosis, Rheumatoide Arthritis, Sarkoidose, Skleritis, Sklerodermie, serpiginöse Choroiditis, Sjogren-Syndrom, Sprue, Sweet-Syndrom, Systemischer Lupus erythematodes, systemische Sklerose, Thrombozytopenie, Epidermolysis acuta toxica, Colitis ulcerosa, Uveitis, Weber-Christian-Krankheit, arzneimittelinduzierte Weber-Christian-Pannikulitis, Wegener-Klinger-Granulomatose.

Vorzugsweise wird 2'-Desoxy-2',2'-difluorcytidin der Formel II 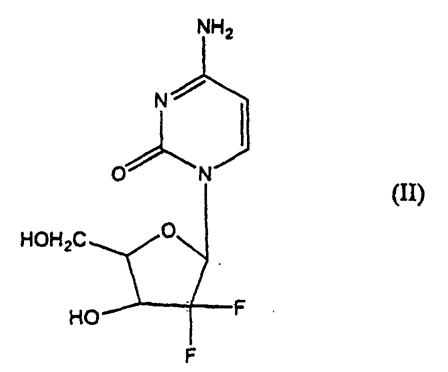 oder ein pharmazeutisch annehmbares Salz davon als Verbindung für obige Verwendungen eingesetzt. Vorzugsweise ist das eingesetzte, pharmazeutisch anwendbare Salz das Hydrochlorid.

Die vorliegende Erfindung ist auch auf die Verwendung von Gemcitabinhydrochlorid in Kombination mit einem oder mehreren von Cyclosporin A, Tacrolimus, Mycophenolatmofetil, Daclizumab, Rapamycin und einem oder mehreren Corticosteroid(en) gerichtet.

Die vorliegende Erfindung stellt weiters pharmazeutische Zusammensetzungen zur Verfügung, welche eine Verbindung der Formel I oder ein pharmazeutisch annehmbares Salz davon, eines oder mehrere von Cyclosporin A, Tacrolimus, Mycophenolatmofetil, Daclizumab, Rapamycin und einem oder mehreren Corticosteroid(en) und einen pharmazeutisch annehmbaren Träger, ein Streckmittel oder ein Vehikel dafür umfassen.

Pharmazeutische Produkte, welche eine Verbindung der Formel I oder ein pharmazeutisch annehmbares Salz davon und eines oder mehrere von Cyclosporin A, Tacrolimus, Mycophenolatmofetil, Daclizumab, Rapamycin und einem oder mehreren Corticosteroid(en) in Kombination zur gleichzeitigen, getrennten oder aufeinanderfolgenden Verwendung zur Therapie des menschlichen oder tierischen Körpers enthalten, werden als noch ein weiterer Aspekt der vorliegenden Erfindung zur Verfügung gestellt.

Vorzugsweise wird 2'-Desoxy-2',2'-difluorcytidin der Formel II oder ein pharmazeutisch annehmbares Salz davon als Verbindung für die pharmazeutischen Zusammensetzungen und pharmazeutischen Produkte der vorliegenden Erfindung eingesetzt. Vorzugsweise ist das eingesetzte, pharmazeutisch anwendbare Salz das Hydrochlorid.

### AUSFÜHRLICHE BESCHREIBUNG DER ERFINDUNG

Die bei der vorliegenden Erfindung eingesetzten Verbindungen der Formel I (2',2'-Difluornucleoside) werden vorzugsweise durch Umsetzen eines D-Glyceraldehydketonids mit einem C₁-C₄ Alkylbromdifluoracetat zur Bildung eines Alkyl-3-dioxolanyl-2,2-difluor-3-hydroxypropionats hergestellt. Das Hydroxypropionat wird dann zu einem Lacton hydrolysiert, welches geschützt und reduziert wird, um ein 2-Desoxy-2,2-difluorribose- oder -xylose-Derivat zu ergeben. Die Hydroxygruppe dieser Verbindung wird mit einer austretenden Gruppe versehen, und das resultierende Kohlenhydrat wird mit einer geeigneten Base gekoppelt. Das resultierende geschützte Nucleosid wird schließlich entschützt, um eine Verbindung zur Verwendung bei der vorliegenden Erfindung zu liefern. Einzelheiten eines Verfahrens zur Herstellung solcher Verbindungen zur Verwendung bei der vorliegenden Erfindung sind im US-Patent 5,464,826 beschrieben, welches hierin durch Bezugnahme aufgenommen ist.

Cyclosporin A, Tacrolimus, Mycophenolatmofetil, Daclizumab, Rapamycin and Corticosteroide sind kommerziell erhältlich.

Die pharmazeutischen Zusammensetzungen und Produkte die in der vorliegenden Erfindung verwendet werden, sind pharmazeutische Formulierungen, welche den Wirkstoff (Verbindung der Formel I) und einen pharmazeutischen Träger, Streckmittel oder Vehikel dafür umfassen. Die Formulierung der Zusammensetzungen und Produkte ist konventionell und folgt den üblichen Praktiken pharmazeutischer Chemiker.

Der Wirkstoff wird in der Formulierung im Bereich von 1 Gew.-% bis 90 Gew.-% enthalten sein. Der Wirkstoff wird üblicherweise mit einem Träger vermischt sein oder durch einen Träger verdünnt sein oder in einem Träger, welcher in Form einer Kapsel, eines Sachets, Papiers oder eines andereren Behältnisses vorliegen kann, eingeschlossen sein. Wenn der Träger als Streckmittel dient, kann er ein festes, halbfestes oder flüssiges Material sein, welches als Vehikel, Arzneistoffträger oder Medium für den Wirkstoff fungiert. Die Zusammensetzungen und Produkte können daher in Form von Tabletten, Pillen, Pulvern, Pastillen, Sachets, Kachets, Elixieren, Suspensionen, Emulsionen, Lösungen, Sirups, Aerosolen (als Feststoff oder in einem flüssigen Medium), Salben, welche beispielsweise bis zu 10 Gew.-% des Wirkstoffes enthalten, Weich- und Hartgelatinekapseln, Suppositorien, sterilen injizierbaren Lösungen und sterilen abbgepackten Pulvern vorliegen.

Einige Beispiele für geeignete Träger, Vehikel und Streckmittel sind beispielsweise Lactose, Dextrose, Saccharose, Sorbitol, Mannitol, Stärken, Gummiarabikum, Calcium-phosphat, Alginate, Tragant, Gelatine, Calciumsilicat, mikrokristalline Cellulose, Polyvinyl-pyrrolidon, Cellulose, Wasser, Sirup, Methylcellulose, Methyl- und Propylhydroxybenzoate, Talk, Magnesiumstearat und Mineralöl. Die Formulierungen können zusätzlich Gleitmittel, Benetzungsmittel, Emulgier- und Suspendiermittel, Konservierungsmittel, Süßstoffe oder Aromastoffe einschließen. Die Zusammensetzungen und Produkte der Erfindung können durch Anwendung bekannter Methoden so formuliert werden, daß sie eine rasche oder Depot-Freisetzung des Wirkstoffes nach Verabreichung an den Patienten oder das Tier bereitstellen.

Die zu verwendenden Zusammensetzungen und Produkte werden vorzugsweise in einer Dosiseinheitsform formuliert, wobei jede Dosis von etwa 0,1 bis etwa 100 mg des Wirkstoffes enthält. Der Begriff "Dosiseinheitsform" bezieht sich auf physikalisch getrennte Einheiten, welche als Dosiseinheiten für Menschen und andere Säugetiere geeignet sind, wobei jede Einheit eine vorbestimmte Menge an Wirkstoff, welche daraufhin berechnet ist, die gewünschte therapeutische Wirkung hervorzurufen, in Verbindung mit einem geeigneten pharmazeutischen Träger enthält.

Wenn der Wirkstoff Gemcitabinhydrochlorid ist, reicht die Dosiseinheit vorzugsweise von etwa 0,5 bis etwa 25 mg und stärker bevorzugt von etwa 1 mg bis etwa 15 mg. Es ist besonders bevorzugt, daß die Dosiseinheit von Gemcitabinhydrochlorid von etwa 1 bis etwa 10 mg und am meisten bevorzugt von etwa 2 mg bis etwa 5 mg reicht.

Die folgenden Formulierungsbeispiele stellen spezifische pharmazeutische Formulierungen dar, für welche als Wirkstoff teilweise Gemcitabinhydrochlorid eingesetzt wird. Für die Formulierungen kann als Wirkstoffjede Verbindung der Formel I eingesetzt werden. Die Beispiele dienen nur der Veranschaulichung und sollen den Bereich der Erfindung in keiner Weise einschränken.

### Formulierung 1

Hartgelatinekapseln werden unter Verwendung der folgenden Bestandteile hergestellt, wobei "Wirkstoff" eine Verbindung der Formel I ist:

| | Menge (mg/Kapsel) |
|---|---|
| Wirkstoff | 25 |
| Getrocknete Stärke | 425 |
| Magnesiumstearat | 10 |

Die oben angeführten Bestandteile werden gemischt und in Mengen zu 460 mg in Hartgelatinekapseln abgefüllt.

### Formulierung 2

Eine Tablettenformulierung wird unter Verwendung der unten angeführten Bestandteile hergestellt:

| | Menge(mg/Tablette) |
|---|---|
| Wirkstoff | 2 |
| Mikrokristalline Cellulose | 500 |
| Siliciumdioxid (abgeraucht) | 10 |
| Sterarinsäure | 8 |

Die Bestandteile werden vermischt und gepreßt, um Tabletten mit einem Gewicht von jeweils 520 mg zu bilden

### Formulierung 3

Eine Aerosollösung wird hergestellt, welche die folgenden Bestandteile enthält:

| | Gewichts-% |
|---|---|
| Wirkstoff | 0.10 |
| Ethanol | 29.90 |
| Treibmittel 22 (Chlordifluormethan) | 70.00 |

Der Wirkstoff wird mit Ethanol gemischt, und das Gemisch wird einem Teil des Treibmittels 22 zugesetzt, auf-30°C gekühlt und in eine Abfülleinrichtung überführt. Die erforderliche Menge wird dann in einen Edelstahlbehälter eingebracht und mit dem Rest des Treibmittels verdünnt. Dann werden die Ventileinheiten am Behälter montiert.

### Formulierung 4

Tabletten, welche jeweils 5 mg Wirkstoff enthalten, sind wie folgt zusammengesetzt:

| | |
|---|---|
| Wirkstoff | 5 mg |
| Stärke | 75 mg |
| Mikrokristalline Cellulose | 58 mg |
| Polyvinylpyrrolidon (als 10%ige Lösung in Wasser) | 5 mg |
| Natriumcarboxymethylstärke | 5.5 mg |
| Magnesiumstearat | 0.5mg |
| Talk | 1 mg |

Der Wirkstoff, Stärke und Cellulose werden durch ein Sieb Nr. 45 mesh U.S. passiert und gründlich vermischt. Die Polyvinylpyrrolidonlösung wird mit den resultierenden Pulvern vermischt, welche dann durch ein Sieb Nr. 14 mesh U.S. passiert werden. Die so produzierten Granula werden bei 50°-60°C getrocknet und durch ein Sieb Nr. 18 mesh U.S. passiert. Dann werden die Natriumcarboxymethylstärke, Magnesiumstearat und Talk, welche vorher durch ein Sieb Nr. 60 mesh U.S. passiert worden sind, den Granula zugesetzt, welche nach dem Mischen in einer Tablettenmaschine verpreßt werden, um Tabletten mit einem Gewicht von jeweils 150 mg zu ergeben.

### Formulierung 5

Kapseln, welche jeweils 0,5 mg Wirkstoff enthalten, werden wie folgt hergestellt:

| | |
|---|---|
| Wirkstoff | 0.5 mg |
| Stärke | 98.5 mg |
| Mikrokristalline Cellulose | 98.5 mg |
| Magnesiumstearat | 2.5 mg |

Der Wirkstoff, Cellulose, Stärke und Magnesiumstearat werden vermischt, durch ein Sieb Nr. 45 mesh U.S. passiert und in Mengen zu 200 mg in Hartgelatinekapseln abgefüllt.

### Formulierung 6

Suppositorien, welche jeweils 0,1 mg Wirkstoff enthalten, werden wie folgt hergestellt:

| | |
|---|---|
| Wirkstoff | 0.1 mg |
| Glyceride gesättigter Fettsäuren auf | 2 g |

Der Wirkstoff wird durch ein Sieb Nr. 60 mesh U.S. passiert und in den vorher unter Anwendung der minimal notwendigen Erwärmung geschmolzenen Glyceriden gesättigter Fettsäuren suspendiert. Das Gemisch wird dann in eine Suppositoriumsform mit einer nominellen Kapazität von 2 g gegossen und abkühlen gelassen.

### Formulierung 7

Suspensionen, welche jeweils 10 mg Wirkstoff pro 5-ml-Dosis enthaiten, werden wie folgt hergestellt:

| | |
|---|---|
| Wirkstoff | 10 mg |
| Natriumcarboxymethylcellulose | 50 mg |
| Sirup | 1.25 ml |
| Benzoesäurelösung | 0.10 ml |
| Aroma | q.v. |
| Farbe | q.v. |
| Gereinigtes Wasser auf | 5 ml |

Der Wirkstoff wird durch ein Sieb Nr. 45 mesh U.S. passiert und mit der Natriumcarboxymethylcellulose und Sirup gemischt, um eine glatte Paste zu bilden. Die Benzoesäurelösung, Aroma und Farbe werden mit einem Teil des Wassers verdünnt und unter Rühren zugesetzt. Dann wird ausreichend Wasser zugesetzt, um das benötigte Volumen zu erzielen.

Intravenöse Formulierungen werden wie folgt hergestellt:

### Formulierung 8

| | |
|---|---|
| Gemcitabin-HCl | 0.1 mg |
| Isotone Kochsalzlösung | 1000 ml |

### Formulierung 9

| | |
|---|---|
| Gemcitabin-HCl | 0.5 mg |
| Isotone Kochsalzlösung | 1000 ml |

### Formulierung 10

| | |
|---|---|
| Gemcitabin-HCl | 1.0 mg |
| Isotone Kochsalzlösung | 1000 ml |

### Formulierung 11

| | |
|---|---|
| Gemcitabin-HCl | 5 mg |
| Isotone Kochsalzlösung | 1000 ml |

### Formulierung 12

| | |
|---|---|
| Gemcitabin-HCl | 10 mg |
| Isotone Kochsalzlösung | 1000 ml |

### Formulierung 13

| | |
|---|---|
| Gemcitabin-HCl | 15 mg |
| Isotone Kochsalzlösung | 1000 ml |

### Formulierung 14

| | |
|---|---|
| Gemcitabin-HCl | 25 mg |
| Isotone Kochsalzlösung | 1000 ml |

Die Lösung der oben angeführten Bestandteile wird intravenös mit einer Geschwindigkeit von beispielsweise 1 ml/Minute verabreicht.

Die Zusammensetzungen und Produkte der Erfindung können dem menschlichen oder tierischen Körper auf verschiedenen Wegen, einschließlich des oralen, rektalen, transdermalen, subkutanen, intravenösen, intramuskulären oder intranasalen Wegs, verabreicht werden.Wenn der Wirkstoff Gemcitabinhydrochlorid ist, wird es vorzugsweise über den IV-Weg verabreicht.

Die Tagesdosen werden normalerweise in einem Bereich von etwa 0,01 bis etwa 10 mg/kg Körpergewicht (KG) - als Einzeldosis oder geteilte Dosen - liegen. Vorzugsweise reichen die Tagesdosen von etwa 0.025 bis etwa 5 mg/kg und am meisten bevorzugt von etwa 0.05 bis 0.25 mg/kg. Es wird jedoch verstanden werden, daß die tatsächlich verabreichte Menge einer Verbindung durch einen Arzt im Lichte der relevanten Begleitumstände, welche den zu behandelnden Zustand, die besondere zu verabreichende Verbindung, den gewählten Verabreichungsweg, das Alter, Gewicht und Ansprechen des einzelnen Patienten und den Schweregrad der Symptome des Patienten einschließen, bestimmt werden wird, und die oben angeführten Dosierungsbereiche sollen daher den Bereich der Erfindung in keiner Weise beschränken.

Die immunsuppressive Wirkung einer repräsentativen Verbindung der Formel I, 2'-Desoxy-2',2'-difluorcytidin (Gemcitabin, dFdC), wurde durch die unten beschriebenen in-vitro- und in-vivo-Untersuchungen gezeigt. Die Verwendung von dFdC stellt lediglich eine bevorzugte Ausführungsform der Erfindung dar, und soll den Bereich der Erfindung in keiner Hinsicht beschränken und auch nicht so ausgelegt werden.

dFdC ist ein Pyrimidinantimetabolit mit antineoplastischer Aktivität gegen eine -große Zahl solider Tumoren, einschließlich des metastasierten Prankreaskarzinoms, des nichtkleinzelligen Bronchialkarzinoms, des Ovarial- und des Mammakarzinoms (Kaye, *J*. *Clin. Oncol*. 12, 1527 (1994)). Es ist ein Desoxycytidin-Analog, welches bei Eintritt in die Zelle durch Desoxycytidinkinase schrittweise zum entsprechenden Di- und Triphosphat als Endprodukt phosphoryliert wird (Plunkett et al., *Nucleosides Nucleotides* 8,775 (1989)). Als Hauptmechanismus wird der Einbau des dFdC-Triphosphates in DNA angenommen, da er die Inhibierung der DNA-Synthese und den Zelltod bewirkt.

Mehrere Phase-I-Studien wurden mit dFdC als Antitumormittel durchgeführt, und die meiste Erfahrung wurde bei Phase-II-Studien mit der wöchentlichen Verabreichung gewonnen (Kaye, *J*. *Clin. Oncol.* 12, 1527 (1994)). Bei diesem Behandlungsschema wird dFdC über 30 Minuten intravenös einmal pro Woche für 3 Wochen verabreicht, gefolgt von einer einwöchigen Ruhepause. Es wird berichtet, daß diese Art der Verabreichung eine Knochenmarksuppression mit schweren Infektionen (WHO-Grad III/IV) bei weniger als 1% der Patienten hervorruft. Sogar nach wiederholten Verabreichungen von dFdC wurde keine signifikante Reduktion der CD4+- and CD8+-Lymphozytenuntergruppen, d.h. keine signifikante Immunsuppression, gefunden (Dalkeler et al., *Anti-Cancer Drugs* 8, 643 (1997)). Dagegen ist die Behandlung niedrigmaligner Lymphome mit einem Purinanalog wie 2-Chlordesoxyadenosin (Cladribin, 2-CdA) mit einem (Täglich x 5)-Schema mit einer schweren Suppression der CD4+-Lymphozyten für mehr als 12 Monate verbunden (Seymour et al., *Blood* 83, 2906 (1994)).

Phase-I-Studien, bei welchen dFdC mit einem (Täglich x 5)-Schema bei einem Dosierungsniveau von 9 mg/m² untersucht wurde, bewirkten ein signifikantes Ausmaß an nicht-hämatologischer Toxizität, einschließlich sporadischem Fieber und schwerer Hypertonie (O'Rourke et al., *Eur*. *J. Cancer* 30A, 417 (1994)). Aufgrund dieser Ergebnisse wurde dieses Schema nicht für eine weitere Evaluierung empfohlen. Messungen der intrazellulären dFdC-Akkumulation nach täglicher Verabreichung niedriger Dosen und deren Effekt auf immunkompetente Zellen sind bisher nicht durchgeführt worden.

Zum Zwecke der vorliegenden Erfindung wurde die immunsuppressive Wirkung von dFdC bewertet, indem der in-vitro-Effekt von dFdC auf Lymphozyten unter Verwendung des Lymphozyten-Koloniewachstumstests und die Wirkung von dFdC in einem Ratten-Herztransplantationsmodell untersucht wurden.

### - Wirkung von dFdC auf die Koloniebildung von T-Lymphozyten

dFdC ist kommerziell erhältlich. Es ist bekannt, daß die Interferenz von Arzneimitteln mit dem Koloniebildungsvermögen aktivierter T-Lymphozyten ein akzeptables Instrument ist, um lymphozytotoxische Wirkungen aufzuzeigen (Aye, *Blood 58, 1043* (1981)). Daher wurden mononukleäre Zellen aus peripherem Blut (PBMC) mit Phytohämagglutinin (PHA) und verschiedenen dFdC-Konzentrationen im von Petzer et al., *Blood* 78,2583 (1991) beschriebenen Mikroagar-Kultursystem kultiviert. Die PBMC wurden in Iscove-Medium, das 20% fötales Kalbsserum und 0,3% Agar enthielt, suspendiert. Anschließend wurden 250-µl-Teilmengen dieser Suspension, welche 2 x 10⁵ PBMC enthielten, auf Gewebskulturplatten mit Vielfachmulden ausplattiert. Der Agar wurde bei Raumtemperatur erstarren gelassen und dann mit 250 µl Medium, das 0.5% PHA and 0.5% 2-Mercaptoethanol (1 x 10⁻⁴ mol/1 Endkonzentrationen) enthielt, überlagert. Die Kulturen wurden bei 37°C in einer 5% CO₂ enthaltenden vollbefeuchteten Atmosphäre inkubiert und die Kolonien wurden unter Verwendung eines umgekehrten Mikroskops nach 7 Tagen Inkubation gezählt.

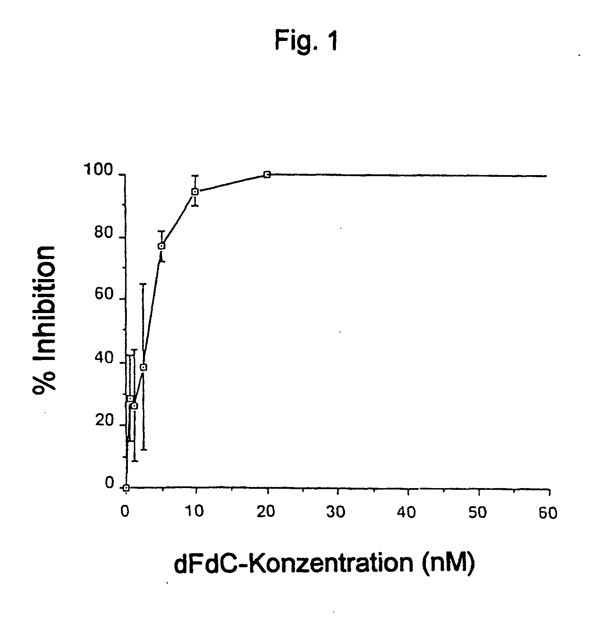

Wie in Fig. 1 gezeigt, wird die PHA-induzierte Lymphozytenproliferation durch dFdC in dosisabhängiger Weise gehemmt, wobei eine 50%ige Hemmung bei einer Konzentration von 3.25 ± 0.9 nmol/l auftritt.

### - Wirkungen von dFdC im Ratten-Herztransplantationsmodell

Durch Inzucht erzeugte männliche LEWIS-(LEW)-Ratten und Brown-Norway-(BN)-Ratten mit einem Gewicht von 200-270 g wurden vom "Zentralinstitut für Versuchstierzucht", Hannover, Deutschland, erhalten. Heterotope Herztransplantate wurden unter Verwendung mikrochirurgischer Techniken, wie von Schmid et al., *Eur*. *Surg. Res.* 30, 61 (1998) beschrieben, durchgeführt. Postoperativ wurde allen Tieren Wasser und Ratten-Standardemährung nach Belieben verabreicht.

dFdC wurde subkutan einmal täglich über 50 aufeinanderfolgende Tage verabreicht, wobei unmittelbar nach dem chirurgischen Eingriff begonnen wurde. Die Tagesdosen (Zahl der Tiere pro Gruppe) betrugen 25 (n=6), 50 (n=5), 75 (n=6), 100 (n=6), 125 (n=6), 150 (n=6), 300 (n=6), 600 (n=2) oder 6000 (n=1) µg/kg Körpergewicht (KG). Die Kontrollgruppe (n=8) erhielt keine Behandlung.

Die Schlagaktivität der Herztransplantate wurde durch tägliche Palpation bestimmt. Wenn die Herztransplantate zu schlagen aufhörten, wurden die Tiere durch eine Überdosis Äther getötet und die Herzen und alle lebenswichtigen Organe für Histologiezwecke entnommen. Multiple Schnitte des linken Ventrikels des Transplantats und jedes nativen Organs wurden mit 4%igem gepufferten Formalin fixiert. In Paraffin eingebettete Proben wurden in 5 µm dicke Schnitte geschnitten und mit Hämatoxylin und Eosin gefärbt. Die Präparate wurden von einem gegenüber der Studie geblindeten Pathologen beurteilt, und die Abstoßung wurde gemäß den ISHT-Kriterien eingestuft (Billingham et al., *J*. *Heart Transplant 9,* 587 (1990)).

Die Wirkungen von dFdC im Ratten-Herztransplantationsmodell sind in Tabelle 1 gezeigt. Die Ergebnisse sind als Transplantatüberlebenszeit in Tagen für die verschiedenen dFdC-Dosisgruppen und die Kontrollgruppe, welche keine dFdC-Behandlung erhielt, dargestellt.

Eine dosisabhängige Leukopenie trat bei allen Tieren aller Gruppen auf und war bei Tieren, die weniger als 150 µg/kg dFdC erhalten hatten, reversibel.

**Tabelle 1**

| dFdC-Dosis (µg/kg/Tag) | Transplantatüberlebenszeit (Tage) | Tränsplantatabstoßung |
|---|---|---|
| Unbehandelte Kontrollen | 7.1 | Grad IV |
| 25 | 7.3 | Grad IV |
| 50 | 9.2 | Grad IV |
| 75 | 15.7 | Grad IV |
| 100 | 152.8 | Grad IV |
| 125 | 144.2 | Grad IV |
| 150 | 41.5 | Keine |
| 300 | 16.0 | Keine |
| 600 | 10.5 | Keine |
| 6000 | 4.0 | Keine |

Die Ergebnisse der oben dargestellten Studien zeigen erstmals eine bemerkenswerte immunsuppressive Wirksamkeit von dFdC. Die Transplantatüberlebenszeit war bei allen Tieren, welchen zwischen 75 und 600 µg/kg KG des Arzneimittels verabreicht worden war, im Vergleich zu unbehandelten Kontrolltieren verlängert. Die längste Überlebenszeit wurde mit 100 bis 125 µg/kg KG erzielt. Mehr als 125 µg/kg KG bewirkten eine Über-Immunsuppression und irreversible Knochenmarktoxizität.

Die durch die oben angeführten Ergebnisse gezeigte wirksame immunsuppressive dFdC-Dosis ist überraschenderweise viel niedriger, als im Hinblick auf die bei der Behandlung maligner Erkrankungen benötigten Dosen des Arzneimittels zu erwarten gewesen wäre.

## Patentansprüche

1. Verwendung einer Verbindung der Formel I 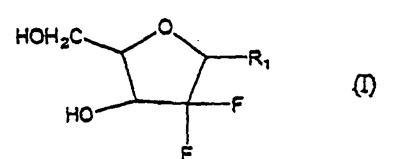 worin R₁ eine durch eine der Formeln 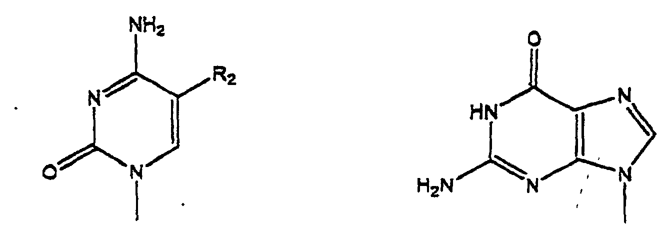 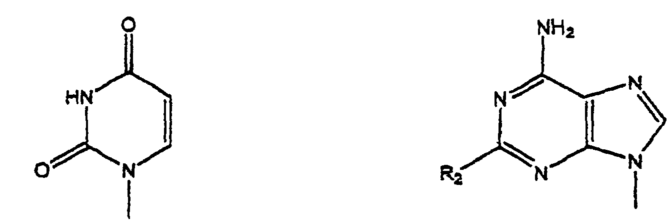 definierte Base ist und R₂ Wasserstoff, C₁-C₄ Alkyl, Brom, Fluor, Chlor oder Iod ist, oder eines pharmazeutisch annehmbaren Salzes davon zur Herstellung eines Medikaments zur immunsuppressiven Therapie des menschlichen oder tierischen Körpers.

2. Verwendung gemäß Anspruch 1 zur Herstellung eines Medikaments zur Behandlung von Autoimmunerkrankungen bei Menschen und Tieren.

3. Verwendung gemäß Anspruch 1 zur Herstellung eines Medikaments zur Unterdrückung der Abstoßung von Transplantaten bei Menschen und Tieren.

4. Verwendung gemäß Anspruch 1 zur Herstellung eines Medikaments zur Behandlung einer Krankheit oder eines Zustands, welche(r) ausgewählt ist aus: Rosacea, Acrodermatitis continua, Aktinisches Retikuloid, Alopezie, Alport-Syndrom, amyotrophische Lateralsklerose, Stomatitis aphthosa, Pure red cell aplasia, aplastische Anämie, Asthma, atopische Dermatitis, Autoimmun-Enteropathie, Behcel-Krankheit, bullöses Erythema exsudativum multiforme, bullöses Pemphigoid, Biliäre Zirrhose, Cornea-Schmelzsyndrom (engl.: corneal melting syndrome), Crohn-Krankheit, Dermatitis herpetiformis, Dermatomyositis, Diabetes mellitus, Duchenne-Form der Muskelatrophie, Ekzem, Epidermolysis bullosa, Erythema nodosum leprosum, familiäre hämophagozytische Lymphohistiozytose, Felty-Syndrom, Granuloma anulare, Grave-Ophthalmopathie, hämolytische Anämie, Hämophilie, Hepatitis, Ichthyosis, entzündliche Erkrankung des Darms (engl.: inflammatory bowel disease), interstitielle Cystitis, interstitielle Lungenkrankheit, Keratokonjunktivitis, Histiozytose der Langerhans-Zellen, Lichen planus, Makrophagenaktivierungssyndrom, Mooren-Ulcus, Morphaea, multiple Sklerose, Myasthenia gravis, Nephropathie, nephrotisches Syndrom, Pustulosis palmaris et plantaris, Pemphigus, persistierende Photosensibilität, Pityriasis rubra pilaris, Polymyositis, Psoriasis, Arthritis psoriatica, Lungenfibrose, Pyoderma gangraenosum, retikuläre erythematöse Mucinosis, Rheumatoide Arthritis, Sarkoidose, Skleritis, Sklerodermie, serpiginöse Choroiditis, Sjogren-Syndrom, Sprue, Sweet-Syndrom, Systemischer Lupus erythematodes, systemische Sklerose, Thrombozytopenie, Epidermolysis acuta toxica, Colitis ulcerosa, Uveitis, Weber-Christian-Krankheit, arzneimittelinduzierte Weber-Christian-Parmikulitis, Wegener-Klinger-Granulomatose.

5. Verwendung gemäß Anspruch 3 zur Unterdrückung der Abstoßung von Knochenmarktransplantaten, Herztransplantaten, Hornhauttransplantaten, Dünndarmtransplantaten, Lebertransplantaten, Lungentransplantaten, Pankreastransplantaten, Nierentransplantaten und Hauttransplantaten.

6. Verwendung gemäß einem oder mehreren der Ansprüche 1 bis 5, wobei die Verbindung der Formel I 2'-Desoxy-2',2'-difluorcytidin der Formel II 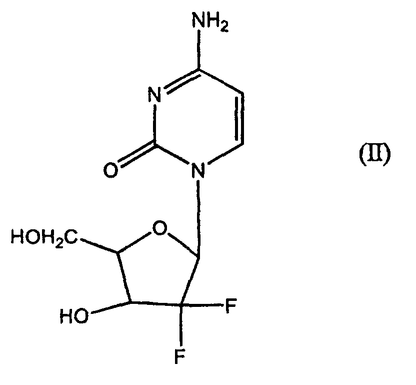 oder ein pharmazeutisch annehmbares Salz davon ist.

7. Verwendung gemäß Anspruch 6, wobei das pharmazeutisch annehmbare Salz das Hydrochlorid ist

8. Verwendung gemäß Anspruch 7, wobei das Gemcitabinhydrochlorid mit einem oder mehreren von Cyclosporin A, Tacrolimus, Mycophenolatmofetil, Daclizumab, Rapamycin und einem oder mehreren Corticosteroid(en) kombiniert ist.

9. Pharmazeutische Zusammensetzung, welche umfaßt:
- eine Verbindung der in Anspruch 1 genannten Formel I oder ein pharmazeutisch annehmbares Salz davon,
- eines oder mehrere von Cyclosporin A, Tacrolimus, Mycophenolatmofetil, Daclizumab, Rapamycin und einem oder mehreren Corticosteroid(en) und
- einen pharmazeutisch annehmbaren Träger, ein Streckmittel oder ein Vehikel dafür.

10. Pharmazeutische Zusammensetzung gemäß Anspruch 9, worin die Verbindung der Formel I 2'-Desoxy-2',2'-difluorcytidin der in Anspruch 6 genannten Formel II oder ein pharmazeutisch annehmbares Salz davon ist.

11. Pharmazeutische Zusammensetzung gemäß Anspruch 10, worin das pharmazeutisch annehmbare Salz das Hydrochiorid ist.

12. Pharmazeutisches Product, welches eine Verbindung der in Anspruch 1 genannten Formel I oder ein pharmazeutisch annehmbares Salz davon und eines oder mehrere von Cyclosporin A, Tacrolimus, Mycophenolatmofetil, Daclizumab, Rapamycin und einem oder mehreren Corticosteroid(en) in Kombination zur gleichzeitigen, getrennten oder aufeinanderfolgenden Verwendung bei der Therapie des menschlichen oder tierischen Körpers enthält.

13. Pharmazeutisches Produkt gemäß Anspruch 12, worin die Verbindung der Formel I 2'-Desoxy-2',2'-difluorcytidin der in Anspruch 6 genannten Formel II oder ein pharmazeutisch annehmbares Salz davon ist.

14. Pharmazeutisches Produkt gemäß Anspruch 13, worin das pharmazeutisch annehmbare Salz das Hydrochlorid ist.

## Claims

1. Use of a compound of the formula I 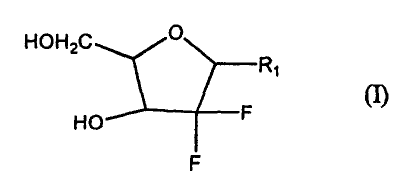 wherein R₁ is a base defined by one of the formulae 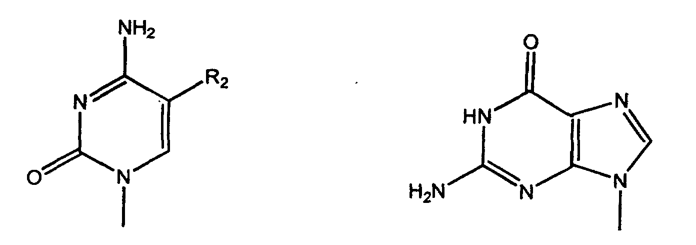 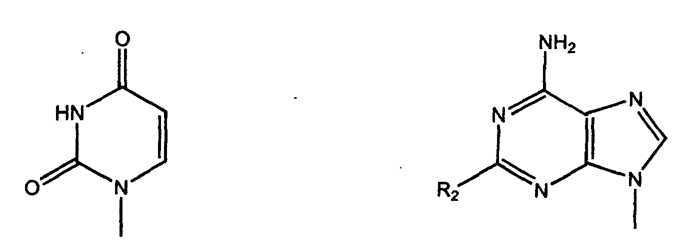 and R₂ is hydrogen, C₁-C₄ alkyl, bromo, fluoro, chloro or iodo; or a pharmaceutically-acceptable salt thereof for the manufacture of a medicament for immunosuppressive therapy of the human or animal body.

2. Use according to claim 1 for the manufacture of a medicament for treating auto-immune diseases in humans and animals.

3. Use according to claim 1 for the manufacture of a medicament for suppressing rejection of transplants in humans and animals.

4. Use according to claim 1 for the manufacture of a medicament for the treatment of a disease or condition selected from: acne rosacea, acrodermatitis continua, actinic reticuloid, alopecia, Alport's syndrome, amyotrophic lateral sclerosis, aphthous stomatitis, red-cell aplasia, aplastic anemia, asthma, atopic dermatitis, autoimmune enteropathy, Behcet's syndrome, bullous erythema multiforme, bullous pemphigoid, biliary cirrhosis, corneal melting syndrome, Crohn's disease, dermatitis herpetiformis, dermatomyositis, diabetes mellitus, Duchenne muscular dystrophy, eczema, epidermolysis bullosa, erythema nodosum leprosum, familial hemophagocytic lymphohistiocytosis, Felty's syndrome, granuloma annulare, Grave's ophthalmopathy, hemolytic anemia, hemophilia, hepatitis, ichthyosis, inflammatory bowel disease, interstitial cystitis, interstitial lung disease, Keratoconjunctivitis, Langerhans cell histiocytosis, lichen planus, macrophage activation syndrome, Mooren's ulcer, morphea, multiple sclerosis, myasthenia gravis, nephropathy, nephrotic syndrome, palmo-plantar pustulosis, pemphigus; persistent photosensitivity, pityriasis rubra pilaris, polymyositis, psoriasis, psoriatic arthritis, pulmonary fibrosis, pyoderma gangrenosum, reticular erythematous mucinosis, rheumatoid arthritis, sarcoidosis, scleritis, scleroderma, serpiginous choroiditis, Sjogren's syndrome, sprue, Sweet's syndrome, systemic lupus erythematosus, systemic sclerosis, thrombocytopenia, toxic epidermal necrolysis, ulcerative colitis, uveitis, Weber-Christian disease, drug-induced Weber-Christian panniculitis, Wegener granulomatosis.

5. Use according to claim 3 for suppressing rejection of bone marrow transplants, cardiac transplants, cornea transplants, intestine transplants, liver transplants, lung transplants, pancreas transplants, renal transplants and skin transplants.

6. Use according to one or more of claims 1 to 5 wherein said compound of formula I is 2'-deoxy-2',2'-difluorocytidine of the formula II 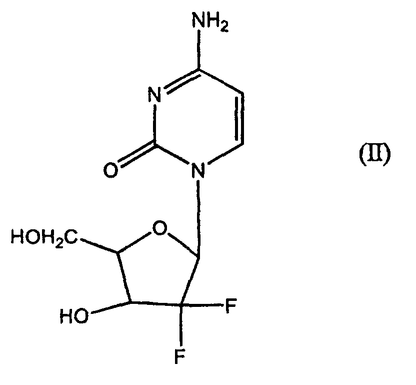 or a pharmaceutically-acceptable salt thereof.

7. Use according to claim 6 wherein said pharmaceutically-acceptable salt is the hydrochloride.

8. Use according to claim 7 wherein the gemcitabine hydrochloride is in combination with one or more of cyclosporin A, tacrolimus, mycophenolate mofetil, daclizumab, rapamycin and one or more corticosteroid(s).

9. A pharmaceutical composition comprising
- a compound of formula I mentioned in claim 1 or a pharmaceutically-acceptable salt thereof;
- one or more of cyclosporin A, tacrolimus, mycophenolate mofetil, daclizumab, rapamycin and one or more corticosteroid(s); and
- a pharmaceutically-acceptable carrier, diluent or excipient therefor.

10. A pharmaceutical composition according to claim 9 in which said compound of formula I is 2'-deoxy-2',2'-difluorocytidine of formula II mentioned in claim 6 or a pharmaceutically-acceptable salt thereof.

11. A pharmaceutical composition according to claim 10 in which said pharmaceutically-acceptable salt is the hydrochloride.

12. A pharmaceutical product containing a compound of formula I mentioned in claim 1 or a pharmaceutically-acceptable salt thereof and one or more of cyclosporin A, tacrolimus, mycophenolate mofetil, daclizumab, rapamycin and one or more corticosteroid(s) in combination for simultaneous, separate or sequential use for therapy of the human or animal body.

13. A pharmaceutical product according to claim 12 in which said compound of formula I is 2'-deoxy-2',2'-difluorocytidine of formula II mentioned in claim 6 or a pharmaceutically-acceptable salt thereof.

14. A pharmaceutical product according to claim 13 in which said pharmaceutically-acceptable salt is the hydrochloride.

## Revendications

1. Utilisation d'un composé de formule I 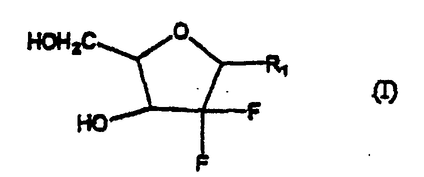 dans laquelle R₁ est une base définie par une des formules 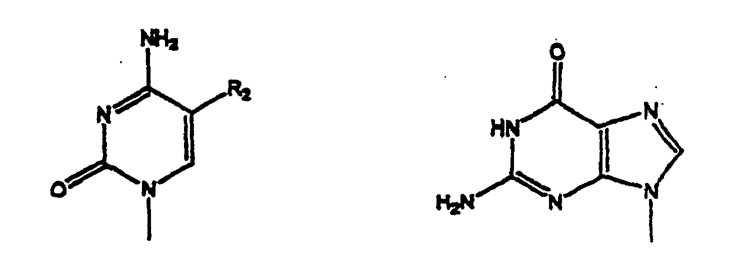 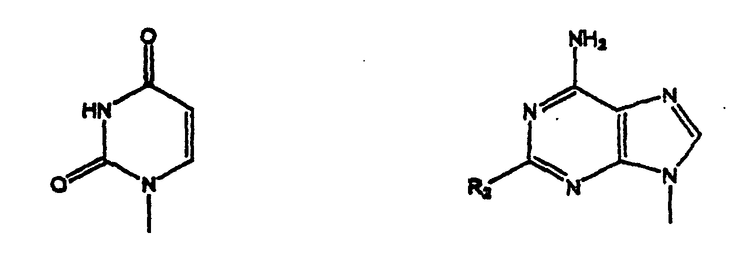 et R₂ est un atome d'hydrogène, un groupe alkyle en C₁ à C₄, un atome de brome, un atome de fluor, un atome de chlore ou un atome d'iode, ou un sel de celui-ci, acceptable sur le plan pharmaceutique, afin de préparer un médicament destiné à la thérapie par immunosuppresseurs du corps humain ou animal.

2. Utilisation selon la revendication 1, pour la préparation d'un médicament destiné au traitement des maladies auto-immunes chez les humains et les animaux.

3. Utilisation selon la revendication 1, pour la préparation d'un médicament destiné à supprimer le rejet de greffons chez les humains et les animaux.

4. Utilisation selon la revendication 1, pour la préparation d'un médicament destiné au traitement d'une maladie ou d'un état, qui est choisi parmi : l'acné rosacée, l'acrodermatite continue, l'actino-réticulose, l'alopécie, le syndrome d'Alport, la sclérose latérale amyotrophique, la stomatite aphteuse, l'érythroblastopénie chronique acquise, l'anémie aplasique, l'asthme, la dermatite atopique, l'entéropathie auto-immune, le syndrome de Behet, l'érythème bulleux, l'érythème exsudatif multiformes, la pemphigoïde bulleuse, la cirrhose biliaire, l'ulcère de Mooren de la cornée (en anglais : corneal melting syndrome), la maladie de Crohn, la dermatite herpétiforme, la dermatomyosite, le diabète sucré, la dystrophie musculaire de Duchenne, l'eczéma, l'épidermolyse bulleuse, l'érythème noueux lépreux, la lymphohistiocytose hémophagocytaire familiale, le syndrome de Felty, le granulome annulaire, l'ophtalmopathie grave, l'anémie hémolytique, l'hémophilie, l'hépatite, l'ichtyose, l'infection inflammatoire du tube digestif (en anglais : inflammatory bowel disease), la cystite interstitielle, la maladie pulmonaire interstitielle, la kératoconjonctivite, l'histiocytose des cellules de Langerhans, le lichen plan, la syndrome d'activation des macrophages, l'ulcère de Mooren, la morphée, la sclérose en plaques, la myasthénie grave, la néphropathie, le syndrome néphrétique, la pustulose palmo-plantaire, un pemphigus, une photosensibilité persistante, le pityriasis rubra pilaire, la polymyosite, le psoriasis, l'arthrite psoriasique, la fibrose pulmonaire, la pyodermite gangréneuse, la mucinose érythémateuse réticulée, la polyarthrite rhumatoïde, la sarcoïdose, la sclérite, la sclérodermie, la choroïdite serpigineuse, le syndrome de Sjogren, la sprue, le syndrome de Sweet, le lupus érythémateux systémique, la sclérose systémique, la thrombocytopénie, l'épidermolyse aiguë toxique, la colite ulcéreuse, l'uvéite, la maladie de Weber-Christian, la panniculite de Weber-Christian induite par les médicaments, la granulomatose de Wegener-Klinger.

5. Utilisation selon la revendication 3, pour supprimer le rejet de greffons de moelle osseuse, de greffons cardiaques, de greffons de cornée, de greffons d'intestin grêle, de greffons de foie, de greffons de poumon, de greffons de pancréas, de greffons de rein, et de greffons de peau.

6. Utilisation selon l'une ou plusieurs des revendications 1 à 5, dans laquelle le composé de formule I est la 2'-desoxy-2',2'-difluorocytidine de formule II 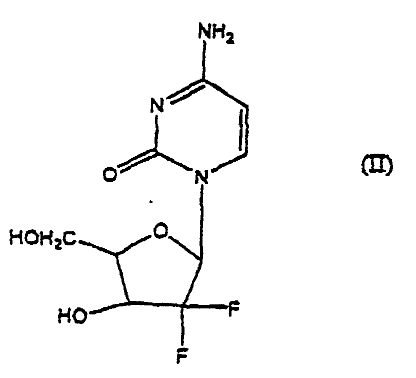 ou un sel de celui-ci, acceptable sur le plan pharmaceutique.

7. Utilisation selon la revendication 6, dans laquelle le sel acceptable sur le plan pharmaceutique est le chlorhydrate.

8. Utilisation selon la revendication 7, dans laquelle le chlorhydrate de gemecitabine est associé à un ou plusieurs médicaments parmi la cyclosporine A, le tacrolimus, le mycophénolatmofétil, le daclizumab, la rapamycine, et un ou plusieurs corticostéroïdes.

9. Composition pharmaceutique, laquelle comprend :
- un composé de formule I citée à la revendication 1, ou un sel de celui-ci, acceptable sur le plan pharmaceutique,
- un ou plusieurs médicaments parmi la cyclosporine A, le tacrolimus, le mycophénolatmofétil, le daclizumab, la rapamycine, et un ou plusieurs corticostéroïdes et
- un support acceptable sur le plan pharmaceutique, un diluant ou un véhicule pour celle-ci.

10. Composition pharmaceutique selon la revendication 9, dans laquelle le composé de formule I est la 2'-desoxy-2',2'-difluorocytidine de formule II citée à la revendication 6 ou un sel de celui-ci, acceptable sur le plan pharmaceutique.

11. Composition pharmaceutique selon la revendication 10, dans laquelle le sel acceptable sur le plan pharmaceutique est le chlorhydrate.

12. Produit pharmaceutique, lequel contient un composé de formule I citée à la revendication 1 ou un sel de celui-ci, acceptable sur le plan pharmaceutique et un ou plusieurs médicaments parmi la cyclosporine A, le tacrolimus, le mycophénolatmofétil, le daclizumab, la rapamycine, et un ou plusieurs corticostéroïde en association en vue d'une utilisation simultanée, séparée ou successive dans la thérapie du corps humain ou animal.

13. Produit pharmaceutique selon la revendication 12, dans lequel le composé de formule I est la 2'-desoxy-2',2'-difluorocytidine de formule II citée à la revendication 6 ou un sel de celui-ci, acceptable sur le plan pharmaceutique.

14. Produit pharmaceutique selon la revendication 13, dans lequel le sel acceptable sur le plan pharmaceutique est le chlorhydrate.
